(19) Europäisches Patentamt / European Patent Office / Office européen des brevets

(11) **EP 3 940 020 A1**

(12) **EUROPEAN PATENT APPLICATION**
published in accordance with Art. 153(4) EPC

(43) Date of publication:
**19.01.2022 Bulletin 2022/03**

(21) Application number: **20772566.4**

(22) Date of filing: **12.03.2020**

(51) International Patent Classification (IPC):
**C08J 3/16** (2006.01)   **A61K 8/85** (2006.01)
**A61K 8/88** (2006.01)   **A61Q 1/12** (2006.01)
**A61Q 19/00** (2006.01)   **C08J 3/03** (2006.01)
**C08L 101/16** (2006.01)

(52) Cooperative Patent Classification (CPC):
**A61K 8/85; A61K 8/88; A61Q 1/12; A61Q 19/00; C08J 3/03; C08J 3/16; C08L 101/16**

(86) International application number:
**PCT/JP2020/010757**

(87) International publication number:
**WO 2020/189485 (24.09.2020 Gazette 2020/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.03.2019   JP 2019048606**
**30.09.2019   JP 2019179492**

(71) Applicant: **Sekisui Kasei Co., Ltd.**
**Kita-ku**
**Osaka-shi**
**Osaka 530-8565 (JP)**

(72) Inventors:
• **MOTOMURA, Takashi**
**Osaka-shi, Osaka 530-8565 (JP)**
• **NISHIUMI, Kengo**
**Osaka-shi, Osaka 530-8565 (JP)**

(74) Representative: **Müller-Boré & Partner**
**Patentanwälte PartG mbB**
**Friedenheimer Brücke 21**
**80639 München (DE)**

(54) **BIODEGRADABLE RESIN PARTICLES, BIODEGRADABLE RESIN PARTICLE GROUP COMPRISING SAID PARTICLES, AND USE THEREOF**

(57) Biodegradable resin particles including a recessed portion in a surface of the particles; and a hollow portion within the particles. The biodegradable resin particles of the present invention can be used in external preparations, such as cosmetics and quasi-drugs; coating materials, such as powder coating compositions and matting agents for coating compositions; rheology modifying agents; anti-blocking agents; smoothing agents; light-diffusing agents; additives for advanced ceramics sintering; fillers for adhesives; and agents for medical diagnosis and examination and can also be used by being added to a resin composition for automotive materials, construction materials, or the like or to a molded product thereof. In particular, the resin particles can be suitably used by being included in an external preparation, examples of which include cosmetics and quasi-drugs; a coating material, examples of which include powder coating compositions and matting agents for coating compositions; or an anti-blocking agent for packaging materials for food and drink or the like.

EP 3 940 020 A1

**Description**

Technical Field

The present invention relates to biodegradable resin particles, a biodegradable resin particle group including the particles, and a use thereof.

Background Art

Resin particles are used for modification and improvement of various materials, with the high specific surface area and a particle structure of the resin particles being utilized. Examples of major uses of resin particles include uses in cosmetic formulations, such as foundations, antiperspirants, and scrubbing agents; uses in various agents, such as matting agents for coating compositions, rheology modifying agents, anti-blocking agents, smoothing agents, light-diffusing agents, and agents for medical diagnosis and examination; and uses in additives for molded products of automotive materials, construction materials, and the like. Examples of the resin particles include urethane particles, acrylic particles, silicone particles, and polyethylene particles.

In this regard, as concerns over environmental issues have been growing in recent years, there is a demand for using a material derived from a non-petroleum raw material or using a biodegradable material in all fields that use a resin, to reduce environmental impact. For example, fields that use resin particles, such as fields of cosmetics and fields of coating compositions, are required to meet this demand.

Known methods for producing biodegradable resin particles include milling methods represented by cryomilling (PTL 1); solvent dissolution-precipitation methods, such as methods in which a resin is dissolved in a solvent at a high temperature, and the resultant is cooled to cause precipitation, and methods in which a resin is dissolved in a solvent, and a poor solvent is subsequently added to cause precipitation (PTL 2 and 3); and methods in which a resin is emulsified at a high temperature with a solvent that does not dissolve resins and also with a large amount of an emulsifying agent (PTL 4).

Citation List

Patent Literature

   PTL 1: Japanese Unexamined Patent Application Publication No. 2017-2291
   PTL 2: International Publication No. WO2012/105140
   PTL 3: Japanese Unexamined Patent Application Publication No. 2005-2302
   PTL 4: International Publication No. WO2017/195642

Summary of Invention

Technical Problem

Unfortunately, in instances where the resin particles of PTL 1 are used in an external preparation, such as a cosmetic, there are problems in that, for example, the resin particles do not have a spherical shape or a small particle diameter; therefore, further improvement is required in the resin particles in terms of spreadability on the skin. The resin particles of PTL 2 to 4 have a relatively spherical shape; however, these resin particles are not sufficient in terms of adhesion to the skin and smooth spreading on the skin, and, therefore, further improvement is required in these resin particles.

An object of the present invention is to provide biodegradable resin particles, a biodegradable resin particle group including the particles, and a use thereof. The biodegradable resin particles are excellent in terms of adhesion to the skin and smooth spreading on the skin.

Solution to Problem

The present invention relates to the following [1] to [6] .

   [1] Biodegradable resin particles including a recessed portion in a surface of the particles; and a hollow portion within the particles.

[2] A biodegradable resin particle group including the biodegradable resin particles according to [1].

[3] An external preparation, including the biodegradable resin particles according to [1] or the biodegradable resin particle group according to [2].

[4] A coating material including the biodegradable resin particles according to [1] or the biodegradable resin particle group according to [2].

[5] A resin composition including the biodegradable resin particles according to [1] or the biodegradable resin particle group according to [2].

[6] An anti-blocking agent including the biodegradable resin particles according to [1] or the biodegradable resin particle group according to [2].

Advantageous Effects of Invention

With the present invention, biodegradable resin particles, a biodegradable resin particle group including the particles, and a use thereof are provided. The biodegradable resin particles are excellent in terms of adhesion to the skin and smooth spreading on the skin. Brief Description of Drawings

[Fig. 1] Fig. 1 is a TEM micrograph of a biodegradable resin particle of Example 1.
[Fig. 2] Fig. 2 is a TEM micrograph of a biodegradable resin particle of Example 4.
[Fig. 3] Fig. 3 is a TEM micrograph of a biodegradable resin particle of Example 7.

Description of Embodiments

(Biodegradable Resin Particles)

Biodegradable resin particles of the present invention (hereinafter also referred to as "resin particles of the present invention") each include at least one recessed portion in a surface of the particle. The inclusion of a recessed portion contributes to excellent adhesion to the skin in instances in which the resin particles are included in an external preparation, such as a cosmetic. A shape of the recessed portion is not particularly limited and may be a spherical shape, an elliptical shape, an irregular shape, or the like.

A major diameter (recessed portion diameter) of an opening portion of the recessed portion may be greater than or equal to 5% of a major diameter of the resin particle, which is preferable from the standpoint of adhesion to the skin; more preferably, the major diameter of the opening portion is greater than or equal to 10%. Furthermore, the major diameter of the opening portion may be less than or equal to 50% of the major diameter of the resin particle, which is preferable from the standpoint of spreadability on the skin; more preferably, the major diameter of the opening portion is less than or equal to 40%, and even more preferably, less than or equal to 30%. The recessed portion diameter and the major diameter of the resin particle can be measured by using a transmission electron microscope (TEM).

A maximum depth of the recessed portion may be greater than or equal to 2% of the major diameter of the resin particle, which is preferable from the standpoint of adhesion to the skin; more preferably, the maximum depth is greater than or equal to 3%. Furthermore, the maximum depth may be less than or equal to 50% of the major diameter of the resin particle, which is preferable from the standpoint of smooth spreading on the skin; more preferably, the maximum depth is less than or equal to 40%, and even more preferably, less than or equal to 30%. The maximum depth of the recessed portion can be measured by using a transmission electron microscope (TEM).

The number of the recessed portions is at least one or more per particle. The number of recessed portions having a recessed portion major diameter of 5 to 50% of the major diameter of the resin particle may be 1 to 10, which is preferable from the standpoint of smooth spreading on the skin: more preferably, the number is 1 to 5. In addition, the number of recessed portions having a recessed portion diameter of 10 to 40% and a maximum depth of the recessed portion of 3 to 40%, of the major diameter of the resin particle, is preferably 1 to 10, more preferably 1 to 5, and even more preferably 1 to 3.

The resin particles of the present invention each include at least one hollow portion within the particle. It is presumed that the mutually independent existence of the recessed portion in the surface of the particle and the hollow portion within the particle contributes to smooth spreading on the skin in instances in which the resin particles are included in an external preparation, such as a cosmetic. A shape of the hollow portion is not particularly limited and may be a spherical shape, an elliptical shape, a needle shape, an irregular shape, or the like.

A major diameter of the hollow portion may be greater than or equal to 10% of the major diameter of the resin particle, which is preferable from the standpoint of smooth spreading on the skin. Furthermore, the major diameter of the hollow portion may be less than or equal to 50% of the major diameter of the resin particle, which is preferable from a similar standpoint: more preferably, the major diameter of the hollow portion is less than or equal to 40%, and even more preferably, less than or equal to 30%. The major diameter of the hollow portion can be measured by using a transmission electron microscope (TEM).

The number of the hollow portions is at least one or more per particle. The number of hollow portions having a major diameter of the hollow portion of 10 to 50% of the major diameter of the resin particle may be 1 to 4, which is preferable from the standpoint of smooth spreading on the skin; more preferably, the number is 1 to 3.

The resin particles of the present invention are not particularly limited provided that the resin particles are biodegradable resin particles. It is preferable that the resin is at least one resin selected from the group consisting of polyester-based resins and polyamide-based resins. Examples of biodegradable polyester-based resins include polybutylene succinate, polybutylene succinate adipate, and polyhydroxy alkanoates. Examples of biodegradable polyamide-based resins include nylon 4. These resins may be used alone or in a combination of two or more.

Among the polyhydroxy alkanoates, poly(3-hydroxyalkanoate) polymers or copolymers including repeating units represented by general formula (1) $[-CH(R)-CH_2CO-O-]$ (where R is an alkyl group represented by $-C_nH_{2n+1}$, and n is an integer of 1 to 15) are preferable. More specifically, it is possible to use a copolymer of 3-hydroxybutyrate and at least one monomer selected from the group consisting of 3-hydroxypropionate, 3-hydroxyvalerate, 3-hydroxyhexanoate, 3-hydroxyheptanoate, 3-hydroxyoctanoate, 3-hydroxynanoate, 3-hydroxydecanoate, 3-hydroxytetradecanoate, 3-hydroxyhexadecanoate, 3-hydroxyoctadecanoate, 4-hydroxybutyrate, 4-hydroxyvalerate, 5-hydroxyvalerate, and 6-hydroxyhexanoate. Specific examples of (3-hydroxyalkanoate) polymers or copolymers include homopolymers of 3-hydroxyalkanoate, copolymers of two or more 3-hydroxyalkanoates with different n values, and mixtures in which two or more selected from the group of the preceding homopolymers and the preceding copolymers are blended together. In particular, a homopolymer, a copolymer, or a mixture including the group consisting of 3-hydroxybutyrate repeating units, in which n is 1; 3-hydroxyvalerate repeating units, in which n is 2; 3-hydroxyhexanoate repeating units, in which n is 3; 3-hydroxyoctanoate repeating units, in which n is 5; and 3-hydroxyoctadecanoate repeating units, in which n is 15 is preferable. A copolymer of 3-hydroxybutyrate repeating units and at least one type of repeating units selected from the group consisting of 3-hydroxyvalerate repeating units, 3-hydroxyhexanoate repeating units, and 3-hydroxyoctanoate repeating units is more preferable.

(Biodegradable Resin Particle Group)

A biodegradable resin particle group of the present invention (hereinafter also referred to as a "resin particle group of the present invention") includes the biodegradable resin particles each of which includes a recessed portion in a surface of the particle and includes a hollow portion. In the resin particle group of the present invention, a proportion of the biodegradable resin particles of the present invention is not particularly limited and preferably as follows: in an instance where a TEM micrograph of randomly selected ten particles having a major diameter within a range of ±50% of a volume average particle diameter is acquired, six or more of the ten particles have a recessed portion and a hollow portion. Furthermore, in the instance where a TEM micrograph of randomly selected ten particles having a major diameter within a range of ±50% of a volume average particle diameter is acquired, an average number of the recessed portions per particle may be 0.5 to 10, and an average number of the hollow portions per particle may be 0.5 to 4; these are preferable from the standpoint of smooth spreading in the skin form. The average number of the recessed portions is more preferably 1 to 5 and even more preferably 1 to 3, and the average number of the hollow portions is more preferably 1 to 3. Note that the recessed portions are recessed portions having a major diameter of 5 to 50% and a maximum depth of 2 to 50%, of the major diameter of the resin particle, and the hollow portions are hollow portions having a major diameter of 10 to 50% of the major diameter of the resin particle.

It is preferable that the resin particle group of the present invention have a volume average particle diameter of 1 to 300 $\mu$m . The volume average particle diameter may be greater than or equal to 1 $\mu$m, which is preferable from the standpoint of the skin feel and smooth spreading in the skin form in instances in which the resin particle group is included in an external preparation, such as a cosmetic (a foundation or an antiperspirant); more preferably, the volume average particle diameter is greater than or equal to 3 $\mu$m, and even more preferably, greater than or equal to 5 $\mu$m. Furthermore, the volume average particle diameter may be less than or equal to 100 $\mu$m, which is preferable from a similar standpoint; more preferably, the volume average particle diameter is less than or equal to 50 $\mu$m, and even more preferably, less than or equal to 30 $\mu$m. On the other hand, in instances where the resin particle group is included in a scrub facial cleanser or the like, the volume average particle diameter may be greater than or equal to 100 $\mu$m, which is preferable from the standpoint of a dirt removal effect; more preferably, the volume average particle diameter is greater than or equal to 150 $\mu$m. Furthermore, the volume average particle diameter may be less than or equal to 300 $\mu$m, which is preferable from a similar standpoint; more preferably, the volume average particle diameter is less than or equal to 250 $\mu$m. The volume average particle diameter is measured by using a method described later in the Examples section.

It is preferable, from the standpoint of adhesion to the skin and smooth spreading on the skin, that the resin particle group of the present invention satisfy the following formula, where $\varphi$ (degree) is an angle of repose, and D ($\mu$m) is the volume average particle diameter. The angle of repose is measured by using a method described later in the

Examples section.

$$-0.97D + 60 \leq \varphi \leq -0.97D + 90$$

The resin particle group of the present invention may have an ash content of less than or equal to 3%, and the ash content is an ash content after the resin particle group is heated at 750°C for 30 minutes. Such an ash content is preferable from the standpoint of adhesion to the skin. The ash content is more preferably less than or equal to 2.5%, even more preferably less than or equal to 2.0%, and still more preferably less than or equal to 1.5%. Furthermore, the ash content may be greater than or equal to 0.1%, which is preferable from the standpoint of the skin feel and smooth spreading in the skin form in instances in which the resin particle group is included in an external preparation, such as a cosmetic; more preferably, the ash content is greater than or equal to 0.2%, and even more preferably, greater than or equal to 0.3%. When the ash content is within the ranges, smooth spreading on the skin can be improved without compromising a moist feel of the particles. The ash content is measured by using a method described later in the Examples section.

The resin particle group of the present invention may have a circularity of greater than or equal to 0.90, which is preferable from the standpoint of smooth spreading in the skin form; more preferably, the circularity is greater than or equal to 0.93, and even more preferably, greater than or equal to 0.95. The upper limit of the circularity may be 1.00 or less, for example. The circularity is measured by using a method described later in the Examples section.

The resin particle group of the present invention may have a mass average molecular weight (Mw) greater than or equal to 10,000, which is preferable from the standpoint of chemical resistance; more preferably, the mass average molecular weight is greater than or equal to 30,000, and even more preferably, greater than or equal to 50,000. Furthermore, the mass average molecular weight may be less than or equal to 1,000,000, which is preferable from the standpoint of good biodegradability for environmental emission; more preferably, the mass average molecular weight is less than or equal to 700,000, and even more preferably, less than or equal to 500,000. The mass average molecular weight is measured by using a method described later in the Examples section.

The resin particle group of the present invention may have a bulk density of greater than or equal to 0.15, which is preferable from the standpoint of adhesion to the skin; more preferably, the bulk density is greater than or equal to 0.20, and even more preferably, greater than or equal to 0.25. Furthermore, the bulk density may be less than or equal to 0.60, which is preferable from the standpoint of smooth spreading on the skin; more preferably, the bulk density is less than or equal to 0.50. The bulk density is measured by using a method described later in the Examples section.

The resin particle group of the present invention may have a thermal weight loss of greater than or equal to 0.05%, which is preferable from the standpoint of the skin feel of the particles and the spreadability on the skin; more preferably, the thermal weight loss is greater than or equal to 0.1%, and even more preferably, greater than or equal to 0.2%. Furthermore, the thermal weight loss may be less than or equal to 3.0%, which is preferable from a similar standpoint; more preferably, the thermal weight loss is less than or equal to 2.5%, and even more preferably, less than or equal to 2.0%. When the thermal weight loss is within the ranges, smooth spreading on the skin can be improved without compromising a moist feel of the particles. The thermal weight loss is measured by using a method described later in the Examples section.

It is preferable, from the standpoint of safety, that the resin particle group of the present invention be free of halogenated solvents. A resin particle group free of halogenated solvents can be obtained by avoiding using a halogenated solvent for the production of the resin particles and the washing of the resin particles. Examples of the halogenated solvents include 1,2-dichloroethane, 1,1-dichloroethane, 1,1,1-trichloroethane, methylene chloride, trans-1,2-dichloroethane, cis-1,2-dichloroethane, chlorobenzene, chloroform, and trichloroethylene. For a method for confirmation of the absence of halogenated solvents, a measurement is to be performed by using a method described later in the Examples section.

The resin particle group of the present invention may further contain a small amount of at least one of 3-alkoxy-3-methyl-1-butanol and 3-alkoxy-3-methyl-1-butyl acetate (the number of carbon atoms of the alkoxy group is 1 to 5). The inclusion of at least one of the substances, which have amphiphilicity, improves affinity for hydrophilic substances and lipophilic substances and also improves adhesion to the skin. A content of the at least one of the substances may be 0.001 to 2 mass%, which is preferable from the standpoint of improving the smooth spreading on the skin and from the standpoint of ease of handling for handling the resin particle group as a powder; more preferably, the content is 0.005 to 1 mass%, and even more preferably, 0.005 to 0.5 mass%. The content of the at least one of the substances is measured by using a method similar to a method described later in the Examples section (a method for measuring a 3-methoxy-3-methyl-1-butanol content).

A method for producing the resin particles of the present invention is carried out as follows. In the presence of a solvent, water, and a dispersion stabilizing agent, a biodegradable resin is emulsified and dispersed at a temperature greater than or equal to 100°C, and subsequently, the resultant is cooled. Accordingly, particles of the biodegradable resin can be obtained. Note that the resin particle group of the present invention can also be obtained by using a similar method.

The solvent may be 3-alkoxy-3-methyl-1-butanol and/or 3-alkoxy-3-methyl-1-butyl acetate (hereinafter also referred to as a "specific solvent"), which are water-miscible. It is preferable, from the standpoint of solubility, that the number of carbon atoms of the alkoxy group in the specific solvent be 1 to 5. Specific examples of such alkoxy groups include methoxy groups, ethoxy groups, propoxy groups, butoxy groups, and pentyloxy groups. The propoxy groups, the butoxy groups, and the pentyloxy groups include not only those having a linear structure but also possible structural isomers. It is preferable that the alkoxy group be a methoxy group, an ethoxy group, or a propoxy group. The specific solvent may be a solvent that is marketed by Kuraray Co., Ltd. under the trade name of Solfit. The 3-alkoxy-3-methyl-1-butanol can be produced, for example, by using a method described in International Publication No. WO2013/146370. The specific solvent is biodegradable and has low skin irritation, and, therefore, in uses in applications such as those for cosmetics, adverse effects that may be caused by a residual solvent can be inhibited. That is, the use of organic solvents having skin irritation (e.g., xylene, toluene, n-methylpyrrolidone, chloroform, methylene chloride, dioxolane, THF, and the like) that are often used in typical micronizing processes for biodegradable resins can be eliminated.

A proportion of the specific solvent in the solvent may be greater than or equal to 50 mass%, which is preferable from the standpoint described above; more preferably, the proportion is greater than or equal to 70 mass%, and even more preferably, 100 mass%. Examples of solvents that may be used in addition to the specific solvent include lower alcohols, such as methanol and ethanol, and acetic acid ester-based solvents, such as ethyl acetate and butyl acetate.

An amount of use of the solvent, per 100 parts by mass of the biodegradable resin, may be greater than or equal to 100 parts by mass, which is preferable from the standpoint of thorough stirring and mixing. Furthermore, the amount may be less than or equal to 1200 parts by mass, which is preferable from the standpoint of productivity; more preferably, the amount is less than or equal to 800 parts by mass, and even more preferably, less than or equal to 400 parts by mass.

The dispersion stabilizing agent may be, for instance, a poorly water-soluble inorganic compound, such as tribasic calcium phosphate (TCP-10U (trade name), manufactured by Taihei Chemical Industrial Co., Ltd.). Among these, tribasic calcium phosphate is preferable in terms of ease of removing the dispersion stabilizing agent. On the other hand, using a water-soluble polymer is not preferable because such a dispersion stabilizing agent tends to remain within the particles and on the surface of the particles and therefore cannot be easily removed. Examples of water-soluble polymers that are not preferable include polyvinyl alcohols, polyvinylpyrrolidones, and celluloses, such as methyl cellulose and ethyl cellulose.

An amount of addition of the dispersion stabilizing agent is preferably 10 to 90 parts by mass, more preferably 15 to 80 parts by mass, and even more preferably 18 to 70 parts by mass, per 100 parts by mass of the biodegradable resin.

Furthermore, a surfactant, such as an anionic surfactant, may also be used, in addition to the dispersion stabilizing agent.

An amount of addition of the surfactant may be 0.01 to 0.5 parts by mass per 100 parts by mass of water.

An amount of use of the water, per 100 parts by mass of the biodegradable resin, may be greater than or equal to 100 parts by mass, which is preferable from the standpoint of thorough stirring and mixing; more preferably, the amount of use is greater than or equal to 150 parts by mass, and even more preferably, greater than or equal to 200 parts by mass. Furthermore, the amount of use may be less than or equal to 2200 parts by mass, which is preferable from the standpoint of productivity; more preferably, the amount of use is less than or equal to 1000 parts by mass, and even more preferably, less than or equal to 800 parts by mass.

Stirring with heating is to be performed at a heating temperature of 100°C or greater so as to achieve micronization. The upper limit of the temperature may be 180°C or less, for example.

To cause a shear force sufficient to form an emulsion during the stirring with heating, the mixing may be performed by using a commonly known method, such as a liquid-phase stirring method that uses a stirring blade, a mixing method that uses a homogenizer, or a ultrasonic irradiation method. The speed and time for the stirring is to be appropriately selected such that the biodegradable resin is uniformly dispersed in the solvent. Typically, the stirring with heating is performed under pressure.

After the stirring with heating, the solvent containing the biodegradable resin is cooled. It is preferable that the cooling from a temperature for the stirring with heating to a cooling temperature be performed gradually. Specifically, it is preferable that the cooling be performed at a rate of 0.5 to 2.0°C/minute. Furthermore, it is preferable that the cooling be performed with stirring. The stirring speed may be within a range similar to that of the stirring speed of the stirring with heating.

After the cooling, acid is added to decompose the dispersion stabilizing agent, and then, filtration, washing, dehydration, and drying are performed. In this manner, the biodegradable resin particles in the solvent are extracted from the solvent. The decomposition of the dispersion stabilizing agent may be performed as follows from the standpoint of the spreadability on the skin and inhibition of hydrolysis: acid is added in an amount 1.05 to 1.50 times the necessary moles, or more preferably 1.05 to 1.20 times the necessary moles, so as to avoid producing strong acid, thereafter, stirring is performed at 40°C or less, and then filtration and washing are performed within 24 hours, or more preferably within 12 hours. The drying can be performed by using a reduced-pressure drying method or a spray drying method.

The dried biodegradable resin particles are to be subjected to classification, and, accordingly, the biodegradable resin particles of the present invention can be obtained. Examples of methods for the classification include air classification and screen classification. The air classification is a method that utilizes an air stream to classify particles. The screen classification is a method in which biodegradable resin particles are fed onto a screen, and the screen is vibrated, so that the biodegradable resin particles on the screen can be separated into particles that pass through the screen mesh and particles that do not pass through the screen mesh.

It is preferable that the classification be performed in a dehumidified air atmosphere so that the biodegradable resin particles do not absorb moisture from the air. Specifically, the classification may be performed preferably in an atmosphere with a relative air humidity of 30% or less, and more preferably in an atmosphere with a relative air humidity of 20% or less. In instances where the classification is performed in a dehumidified air atmosphere, an increase in a moisture content of the biodegradable resin particles is inhibited, which in turn inhibits degradation of the resin due to hydrolysis.

It is preferable that after production, the biodegradable resin particles obtained as described above be hermetically enclosed in a low-moisture-permeability packaging material and stored as a packaged article, so that the biodegradable resin particles do not absorb moisture from the air. Preferably, the low-moisture-permeability packaging material may be a packaging material having a moisture vapor transmission rate of less than or equal to 50 $g/m^2\cdot24$ hours. Examples of such a packaging material include bags made of low-density polyethylene and having a thickness of 50 to 150 $\mu m$; bags made of a deposited film including a synthetic resin film having a surface on which a metal film has been deposited; and bags made of a layered film including a synthetic resin film having a surface on which a metal film has been integrally layered. Regarding the moisture permeability of the packaging material, the moisture vapor transmission rate is preferably less than or equal to 50 $g/m^2\cdot24$ hours and more preferably less than or equal to 30 $g/m^2\cdot24$ hours. The moisture vapor transmission rate was measured in accordance with JIS K 7129 B (2000) (an infrared sensor method) by using a moisture vapor transmission rate transmission rate measuring device Permatran® W3/31, manufactured by Mocon, Inc., the United States, under conditions including a temperature of 40°C and a relative humidity of 90%. For two test specimens, the measurement is performed once per test specimen, and the value of the moisture vapor transmission rate is determined as the arithmetic mean of the two measured values. The use of a low-moisture-permeability packaging material for storage inhibits an increase in the moisture content of the biodegradable resin particles during long-time storage, which in turn inhibits degradation of the resin due to hydrolysis.

The resin particles of the present invention can be used, for example, inexternal preparationsexternal preparations, such as cosmetics and quasi-drugs; coating materials, such as powder coating compositions and matting agents for coating compositions; rheology modifying agents; anti-blocking agents; smoothing agents; light-diffusing agents; additives for advanced ceramics sintering; fillers for adhesives; and agents for medical diagnosis and examination and can also be used by being added to a resin composition for automotive materials, construction materials, or the like or to a molded product thereof. In particular, the resin particles can be suitably used by being included in an external preparation, examples of which include cosmetics and quasi-drugs; a coating material, examples of which include powder coating compositions and matting agents for coating compositions; or an anti-blocking agent for packaging materials for food and drink or the like.

An exemplary embodiment of an external preparation including the resin particle group of the present invention will be described below. The formulation for external use is intended to be used in cosmetics.

A content of the resin particle group of the present invention in a cosmetic may be appropriately set in accordance with the type of the cosmetic. The content in the cosmetic may be greater than or equal to 1 mass%, which is preferable from the standpoint of enabling an effect of the resin particle group of the present invention to be produced; more preferably, the content is greater than or equal to 3 mass%. Furthermore, the content may be less than or equal to 80 mass%, which is preferable from the standpoint of production costs and the like; more preferably, the content is less than or equal to 50 mass%, even more preferably, less than or equal to 30 mass%, and still more preferably, less than or equal to 15 mass%.

Examples of cosmetics of the present invention include, but are not limited to, cleansing cosmetics, such as soaps, body shampoos, facial cleansing creams, scrub facial cleansers, and dentifrices; make-up cosmetics, such as white makeup powders, face powders (e.g., loose powders and pressed powders), foundations (e.g., powder foundations, liquid foundations, and emulsion-type foundations), lipsticks, lip balms, blushers, cosmetics for eyes and eyebrows, and nail polishes; lotions, such as pre-shave lotions and body lotions; external preparations for bodies, such as body powders and baby powders; skin care agents, such as skin lotions, creams, milky lotions (cosmetic milky lotions); antiperspirants (e.g., liquid antiperspirants, solid antiperspirants, and cream antiperspirants); packs; hair-washing cosmetics; hair colors; hair dressings; fragrant cosmetics; bath preparations; sunscreen agents; sun tanning agents; and shaving creams.

Furthermore, the cosmetics of the present invention may include a commonly used base compound or an additive in accordance with a purpose, to an extent that does not impair the effects of the present invention. Examples of the base compound or additive include water, lower alcohols (alcohols having 5 or fewer carbon atoms), fats and oils, waxes, hydrocarbons, higher fatty acids, higher alcohols, sterols, fatty acid esters, metal soaps, moisturizing agents, surfactants, polymers, coloring materials, flavoring agents, clay minerals, antiseptic and bactericidal agents, antiinflammatory agents, antioxidants, UV absorbers, organic-inorganic composite particles, pH adjusting agents (e.g., triethanolamine), specially formulated additives, and pharmaceutical active substances.

Specific examples of the fats and oils and the waxes include avocado oil, almond oil, olive oil, cacao butter, beef tallow, sesame oil, wheat germ oil, safflower oil, shea butter, turtle oil, camellia oil, persic oil, castor oil, grape seed oil, macadamia nut oil, mink oil, egg-yolk oil, Japan wax, coconut oil, rose hip oil, hydrogenated oil, silicone oil, orange roughy oil, carnauba wax, candelilla wax, spermaceti wax, jojoba oil, montan wax, beeswax, and lanolin.

Specific examples of the hydrocarbons include liquid paraffin, petrolatum, paraffin, ceresin, microcrystalline wax, and squalane.

Specific examples of the higher fatty acids include lauric acid, myristic acid, palmitic acid, stearic acid, oleic acid, behenic acid, undecylenic acid, oxystearic acid, linoleic acid, lanolin fatty acid, fatty acids having 11 or more carbon atoms, such as synthetic fatty acid.

Specific examples of the higher alcohols include alcohols having 6 or more carbon atoms, such as lauryl alcohol, cetyl alcohol, cetostearyl alcohol, stearyl alcohol, oleyl alcohol, behenyl alcohol, lanolin alcohol, hydrogenated lanolin alcohol, hexyldecanol, octyldecanol, isostearyl alcohol, jojoba alcohol, and decyltetradecanol.

Specific examples of the sterols include cholesterol, dihydrocholesterol, and phytocholesterol.

Specific examples of the fatty acid esters include linoleic acid esters, such as ethyl linoleate; lanolin fatty acid esters, such as isopropyl lanolate; lauric acid esters, such as hexyl laurate; myristic acid esters, such as isopropyl myristate, myristyl myristate, cetyl myristate, and octyldodecyl myristate; oleic acid esters, such as decyl oleate and octyldodecyl oleate; dimethyl octanoic acid esters, such as hexyldecyl dimethyloctanoate; isooctanoic acid esters, such as cetyl isooctanoate (cetyl 2-ethylhexanoate); palmitic acid esters, such as decyl palmitate; glycerol trimyristate, glycerol tricaprylate/caprate, propylene glycol dioleate, glycerol triisostearate, glycerol triisooctanoate, cetyl lactate, myristyl lactate, diisostearyl malate, and cyclic alcohol fatty acid esters, such as cholesteryl isostearate and cholesteryl 12-hydroxystearate.

Specific examples of the metal soaps include zinc laurate, zinc myristate, magnesium myristate, zinc palmitate, zinc stearate, aluminum stearate, calcium stearate, magnesium stearate, and zinc undecylenate.

Specific examples of the moisturizing agents include glycerol, propylene glycol, 1,3-butylene glycol, polyethylene glycol, sodium dl-pyrrolidonecarboxylate, sodium lactate, sorbitol, sodium hyaluronate, polyglycerol, xylitol, and maltitol.

Specific examples of the surfactants include anionic surfactants, such as higher fatty acid soaps, higher alcohol sulfuric acid esters, N-acyl glutamic acid salts, and phosphoric acid ester salts; cationic surfactants, such as amine salts and quaternary ammonium salts; amphoteric surfactants, such as betaine-type surfactants, amino acid-type surfactants, imidazoline-type surfactants, and lecithin; and nonionic surfactants, such as fatty acid monoglyceride, polyethylene glycol, propylene glycol fatty acid esters, sorbitan fatty acid esters, sucrose fatty acid esters, polyglycerol fatty acid esters, and ethylene oxide condensates.

Specific examples of the polymers include natural polymers, such as gum arabic, gum tragacanth, guar gum, locust bean gum, karaya gum, Irish moss, quince seed, gelatin, shellac, rosin, and casein; semi-synthetic polymers, such as sodium carboxymethyl cellulose, hydroxyethyl cellulose, methyl cellulose, ethyl cellulose, sodium alginate, ester gum, nitrocellulose, hydroxypropyl cellulose, and crystalline cellulose; and synthetic polymers, such as polyvinyl alcohol, polyvinylpyrrolidone, sodium polyacrylate, carboxyvinyl polymers, polyvinyl methyl ether, polyamide resins, silicone oils, and resin particles, such as nylon particles, poly(meth)acrylic acid ester particles (e.g., polymethylmethacrylate particles or the like), polystyrene particles, silicone-based particles, urethane particles, polyethylene particles, and silica particles.

Specific examples of the coloring materials include inorganic pigments, such as iron oxide (e.g., red iron oxide, yellow iron oxide, and black iron oxide), ultramarine blue, iron blue, chromium oxide, chromium hydroxide, carbon black, manganese violet, titanium oxide, zinc oxide, talc, kaolin, calcium carbonate, magnesium carbonate, mica, aluminum silicate, barium silicate, calcium silicate, magnesium silicate, silica, zeolite, barium sulfate, calcined calcium sulfate (calcined gypsum), calcium phosphate, hydroxyapatite, and ceramic powders; and tar dyes, such as azo-based dyes, nitro-based dyes, nitroso-based dyes, xanthene-based dyes, quinoline-based dyes, anthraquinoline-based dyes, indigo-based dyes, triphenylmethane-based dyes, phthalocyanine-based dyes, and pyrene-based dyes.

Note that raw material powders of the polymers and raw material powders of the coloring materials or the like may be ones surface-treated in advance. As the method for the surface treatment, a known surface treatment technique may be used. Examples of the treatment technique include treatment with oil, such as a hydrocarbon oil, an ester oil, or lanolin; treatment with silicone, such as dimethylpolysiloxane, methylhydrogenpolysiloxane, or methylphenylpolysiloxane; treatment with a fluorinated compound, such as a perfluoroalkyl-group-containing ester, a perfluoroalkylsilane, a perfluoropolyether, or a perfluoroalkyl-group-containing polymer; treatment with a silane coupling agent, such as 3-methacryloxypropyl trimethoxysilane or 3-glycidoxypropyltrimethoxysilane; treatment with a titanium coupling agent, such as isopropyl triisostearoyl titanate or isopropyl tris(dioctylpyrophosphate) titanate; treatment with a metal soap; treatment with amino acid, such as acyl glutamic acid; treatment with lecithin, such as hydrogenated egg-yolk lecithin; treatment with collagen; treatment with polyethylene; moisturizing treatment; treatment with an inorganic compound; and mechanochemical treatment.

Specific examples of the clay minerals include ingredients that have multiple functions, such as a function of an extender pigment and a function of an adsorbent, and examples of the ingredients include talc, mica, sericite, titanium sericite (sericite coated with titanium oxide), muscovite, and Veegum®, manufactured by Vanderbilt.

Specific examples of the flavoring agents include anisaldehyde, benzyl acetate, and geraniol.

Specific examples of the antiseptic and bactericidal agents include methylparapen, ethylparapen, propylparapen, benzalkonium, and benzethonium.

Specific examples of the antioxidants include dibutylhydroxytoluene, butylhydroxyanisole, propyl gallate, and tocopherol.

Specific examples of the UV absorbers include inorganic absorbers, such as micronized titanium oxide, micronized zinc oxide, micronized cerium oxide, micronized iron oxide, and micronized zirconium oxide; and organic absorbers, such as benzoic acid-based absorbers, para-aminobenzoic acid-based absorbers, anthranilic acid-based absorbers, salicylic acid-based absorbers, cinnamic acid-based absorbers, benzophenone-based absorbers, and dibenzoylmethane-based absorbers.

Specific examples of the specially formulated additives include hormones, such as estradiol, estrone, ethinyl estradiol, cortisone, hydrocortisone, and prednisone; vitamins, such as vitamin A, vitamin B, vitamin C, and vitamin E; skin astringents, such as citric acid, tartaric acid, lactic acid, aluminum chloride, aluminum potassium sulfate, aluminum chlorohydroxy allantoinate, zinc p-phenol sulfonate, and zinc sulfate; hair growth stimulants, such as cantharides tincture, capsicum tincture, ginger tincture, swertia extract, garlic extract, hinokitiol, carpronium chloride, glyceride pentadecanoate, vitamin E, estrogen, and photosensitive elements; and whitening agents, such as magnesium L-ascorbyl phosphate and kojic acid.

An exemplary embodiment of a coating material including the resin particle group of the present invention will be described below. The coating material includes, if necessary, a binder resin, a UV-curable resin, a solvent, and the like, in addition to the resin particle group of the present invention. The binder resin may be a resin that is soluble in an organic solvent or water or may be an emulsion-type resin that can be dispersed in water.

In this embodiment, examples of the binder resin include biodegradable resins such as polylactic acid, polyglycolic acid, polybutylene succinate, polybucylene succinate adipate, polybutylene adipate terephthalate, poly(ethylene succinate-terephthalate), poly(butylene succinate-terephthalate), poly(butylene adipate-terephthalate), poly($\epsilon$-caprolactone), poly($\beta$-propiolactone), polyamide 4, poly(3-hydroxybutyrate), poly(3-hydroxyvalerate), poly(3-hydroxycaprolate), poly(3-hydroxyheptanoate), poly(3-hydroxyoctanoate), poly(3-hydroxybutyrate-3-hydroxyhexanoate), poly(3-hydroxybutyrate-3-hydroxyvalerate), starch-based resins, cellulose-based resins, and glucosamine-based resins; acrylic resins; alkyd resins; polyester resins; polyurethane resins; biodegradable resins; chlorinated polyolefin resins; and amorphous polyolefin resins.

In this embodiment, examples of the UV-curable resins include multifunctional (meth)acrylate resins, such as polyhydric alcohol multifunctional (meth)acrylate, and multifunctional urethane acrylate resins, such as those synthesized from, for example, diisocyanate, a polyhydric alcohol, and a hydroxy-group-containing (meth)acrylic acid ester. Among these, multifunctional (meth)acrylate resins are preferable, and polyhydric alcohol multifunctional (meth)acrylate resins having three or more (meth)acryloyl groups in one molecule are more preferable. Specifically, examples of the polyhydric alcohol multifunctional (meth)acrylate resins having three or more (meth)acryloyl groups in one molecule include trimethylolpropane tri(meth)acrylate, trimethylolethane tri(meth)acrylate, 1,2,4-cyclohexane tetra(meth)acrylate, pentaglycerol triacrylate, pentaerythritol tetra(meth)acrylate, pentaerythritol tri(meth)acrylate, dipentaerythritol triacrylate, dipentaerythritol pentaacrylate, dipentaerythritol tetra(meth)acrylate, dipentaerythritol hexa(meth)acrylate, tripentaerythritol triacrylate, and tripentaerythritol hexaacrylate. These may be used alone or in a combination of two or more. In instances where a UV-curable resin is used, a photopolymerization initiator may be added to the UV-curable resin to form a binder resin. The photopolymerization initiator is not particularly limited.

In this embodiment, examples of the polymerization initiator include acetophenones, benzoins, benzophenones, phosphine oxides, ketals, $\alpha$-hydroxyalkylphenones, $\alpha$-aminoalkylphenones, anthraquinones, thioxanthones, azo compounds, peroxides (described in Japanese Unexamined Patent Application Publication No. 2001-139663 and others), 2,3-dialkyldione compounds, disulfide compounds, fluoroamine compounds, aromatic sulfonium salts, onium salts, borate salts, active halogen compounds, and $\alpha$-acyloxime esters.

The binder resin or the UV-curable resin may be appropriately selected in consideration of adhesion of the coating composition to a substrate to which the coating composition is to be applied, the usage environment, and the like.

In this embodiment, a content of each of the ingredients described above varies depending on a thickness of the coating film to be formed, the average particle diameter of the resin particle group, and a coating method. An amount of addition of the resin particle group of the present invention is preferably 1 to 50 mass%, more preferably 3 to 45 mass%, and even more preferably 5 to 40 mass%, relative to a total content of the binder resin and the resin particle group of the present invention, which is taken as 100 mass% (in instances where the binder resin is an emulsion-type aqueous resin, the content of the binder is the solids content).

In this embodiment, the solvent is not particularly limited and is preferably a solvent in which the binder resin or the UV-curable resin can be dissolved or dispersed. When the coating material is an oil-based coating composition, examples of the solvent include hydrocarbon-based solvents, such as toluene and xylene; ketone-based solvents, such as methyl ethyl ketone and methyl isobutyl ketone; ester-based solvents, such as ethyl acetate and butyl acetate; and ether-based solvents, such as dioxane, ethylene glycol diethyl ether, and ethylene glycol monobutyl ether. When the coating material is an aqueous coating composition, examples of the solvent include water and alcohols. These solvents may be used alone or in a combination of two or more. A content of the solvent in the coating material is typically approximately 20 to 60 mass% relative to a total mass of the coating composition.

In this embodiment, the coating material may include, if necessary, one or more known agents, examples of which include coating surface modifiers, fluidity modifiers, UV absorbers, light stabilizers, curing catalysts, extender pigments, color pigments, metallic pigments, mica powder pigments, and dyes

In this embodiment, a coating film in which the coating material is used can be formed by using any known method, without limitation. Examples of the methods include spray coating methods, roll coating methods, and brush coating methods. In instances where a thin layer of the coating material is to be coated onto a substrate such as a film, examples of the methods include reverse roll coating methods, gravure coating methods, die coating methods, comma coating methods, and spray coating methods. The coating composition may be diluted if necessary so that a viscosity thereof can be adjusted. Examples of the diluent include hydrocarbon-based solvents, such as toluene and xylene; ketone-based solvents, such as methyl ethyl ketone and methyl isobutyl ketone; ester-based solvents, such as ethyl acetate and butyl acetate; ether-based solvents, such as dioxane and ethylene glycol diethyl ether; water; and alcoholic solvents. These diluents may be used alone or in a combination of two or more. The coating material may be applied to any coating surface of a substrate or the like to produce a coating film, and after the coating film is dried, the coating film may be cured as necessary to form a crosslinked coating film. Note that the coating film in which the coating material is used can serve as a coating for various substrates, examples of which include, but are not limited to, substrates made of metal, wood, glass, or plastic. Furthermore, the coating film may serve as a coating for transparent substrates, examples of which include substrates made of PET, PC, or acrylic.

An exemplary embodiment of an anti-blocking agent including the resin particle group of the present invention will be described below. The biodegradable resin particles of the present invention can be used as an anti-blocking agent that provides irregularities on a surface of a resin film and, accordingly, prevents a phenomenon in which when, for instance, a resin film is wound up, surfaces of the resin film that are in contact with each other adhere to each other and therefore cannot be separated from each other (blocking). In this embodiment, the anti-blocking agent may include, if necessary, one or more known additives in addition to the resin particle group of the present invention. Examples of the additives include antioxidants, fluidity modifiers, light stabilizers, and color pigments.

In this embodiment, a content of the resin particle group of the present invention in the anti-blocking agent is preferably 70 to 100 mass%, more preferably 80 to 100 mass%, and even more preferably 90 to 100 mass%.

In this embodiment, resin films for which the anti-blocking agent can be used are resin films made of a resin, and examples of the resin include biodegradable resins such as polylactic acid, polyglycolic acid, polybutylene succinate, polybucylene succinate adipate, polybutylene adipate terephthalate, poly(ethylene succinate-terephthalate), poly(butylene succinate-terephthalate), poly(butylene adipate-terephthalate), poly($\varepsilon$-caprolactone), poly($\beta$-propiolactone), polyamide 4, poly(3-hydroxybutyrate), poly(3-hydroxyvalerate), poly(3-hydroxycaprolate), poly(3-hydroxyheptanoate), poly(3-hydroxyoctanoate), poly(3-hydroxybutyrate-3-hydroxyhexanoate), poly(3-hydroxybutyrate-3-hydroxyvalerate), starch-based resins, cellulose-based resins, and glucosamine-based resins; polyester-based resins, such as polyethylene terephthalate and polyethylene naphthalate; polyolefin-based resins, such as polyethylene-based resins and polypropylene-based resins; (meth)acrylic-based resins; polystyrene-based resins; polyether sulfone-based resins; polyurethane-based resins; polycarbonate-based resins; polysulfone-based resins; polyether-based resins; polymethylpentene-based resins; polyetherketone-based resins; (meth)acrylonitrile-based resins; norbornene-based resins; amorphous polyolefin-based resins; polyamide resins; polyimide resin; and triacetyl cellulose resins.

In the resin film, a content of the resin particle group of the present invention varies depending on a thickness of the film to be formed, the average particle diameter of the resin particle group, and a molding method. The content of the resin particle group of the present invention is preferably 0.01 to 10 mass%, more preferably 0.01 to 5 mass%, even more preferably 0.01 to 3 mass%, and particularly preferably 0.01 to 1 mass%, in the resin film.

An exemplary embodiment of a resin composition including the resin particle group of the present invention will be described below. In this embodiment, the resin composition includes the resin particle group of the present invention and a base resin. Examples of the base resin include biodegradable resins such as polylactic acid, polyglycolic acid, polybutylene succinate, polybucylene succinate adipate, polybutylene adipate terephthalate, poly(ethylene succinate-terephthalate), poly(butylene succinate-terephthalate), poly(butylene adipate-terephthalate), poly($\varepsilon$-caprolactone),

poly(β-propiolactone), polyamide 4, poly(3-hydroxybutyrate), poly(3-hydroxyvalerate), poly(3-hydroxycaprolate), poly(3-hydroxyheptanoate), poly(3-hydroxyoctanoate), poly(3-hydroxybutyrate-3-hydroxyhexanoate), poly(3-hydroxybutyrate-3-hydroxyvalerate), starch-based resins, cellulose-based resins, and glucosamine-based resins; and thermoplastic resins, such as polycarbonate, polyethylene terephthalate, polybutylene terephthalate, polyamide 6, polyamide 66, polyamide 12, ABS resins (acrylonitrile-butadiene-styrene copolymer resins), AS resins (acrylonitrile-styrene copolymer resins), polyethylene, polypropylene, polyacetal, polyamide-imide, polyethersulfone, polyimide, polyphenylene oxide, polyphenylene sulsalfide, polystyrene, thermoplastic polyurethane elastomers, thermoplastic polyester elastomers, thermoplastic polyamide elastomers, polyvinyl chloride, polyvinylidene fluoride, ethylene-tetrafluoroethylene copolymers (ETFE resins), tetrafluoroethylene-perfluoroalkyl vinyl ether copolymers (PFA resins), and polyetherketone. These resins may be used alone or in a combination of two or more.

In this embodiment, in the resin composition, a content of the resin particle group of the present invention varies depending on a thickness of a molded product to be formed, the average particle diameter of the resin particle group, and a molding method. The content of the resin particle group of the present invention is preferably 0.1 to 70 mass%, more preferably 0.5 to 50 mass%, and even more preferably 1 to 30 mass%, relative to a total content of the base resin and the resin particle group of the present invention, which is taken as 100 mass%.

In this embodiment, the resin composition may include, if necessary, one or more known additives. Examples of the additives include reinforcing fibers, such as glass fibers and carbon fibers, flame retardants, fluidity modifiers, UV absorbers, heat stabilizers, light stabilizers, lubricants, extender pigments, color pigments, metallic pigments, and dyes.

In this embodiment, the resin composition can be produced by mixing the resin particle group with the base resin by using a method known in the art, without limitation. Examples of the method include mechanical grinding and mixing methods. With a mechanical grinding and mixing method, the resin composition can be produced by mixing and stirring the resin particle group with the base resin by using, for example, an apparatus such as a Henschel mixer, a V-type mixer, a Turbula mixer, a hybridizer, or a rocking mixer.

In this embodiment, a molded product in which the resin composition is used can be formed by using any known method, without limitation. For example, a molded product having a shape suitable for automotive materials, construction materials, packaging materials, and the like can be obtained as follows. Pellets made of the resin composition are prepared by mixing the resin particle group of the present invention with a base resin in a mixer and kneading the mixture in a melt kneading apparatus, such as an extrusion apparatus, and subsequently, molding the pellets by extrusion, injection, or blow molding, for example.

EXAMPLES

Now, the present invention will be described in detail with reference to examples. Note that the examples are merely illustrative of the present invention and are not intended to limit the present invention. In the examples, "parts" means "parts by mass", and "%" means "mass%" unless otherwise specified.

<TEM Micrograph Acquisition>

The biodegradable resin particles are embedded (60°C, 24 hours) in an epoxy resin, and an ultrathin section (a thickness of 70 nm) is prepared therefrom by using an ultramicrotome (Leica EM UC7, manufactured by Leica Microsystems). Subsequently, micrographs of the ultrathin section are acquired by using a transmission electron microscope (H-7600, manufactured by Hitachi High-Technologies Corporation, with a CCD camera system ER-B, manufactured by Advanced Microscopy Techniques, Corp.) at an acceleration voltage of 20 kV and a magnification of 500 to 20000 times. The staining agent to be used in the preparation of the ultrathin section is ruthenium tetraoxide.

<Measurement of Volume Average Particle Diameter>

The volume average particle diameter of the biodegradable resin particle group is to be measured by using a Coulter Multisizer™ 3 (manufactured by Beckman Coulter, Inc.). To carry out the measurement, an aperture calibrated in accordance with a user's manual is to be appropriately selected based on a size of the particles to be measured. The measurement sample to be used is a dispersion obtained as follows: 0.1 g of the biodegradable resin particle group is dispersed in 10 m1 of a 0.1 mass% aqueous solution of a nonionic surfactant by using a touch mixer (Touch Mixer MT-31, manufactured by Yamato Scientific Co., Ltd.) and an ultrasonic cleaner (Ultrasonic Cleaner VS-150, manufactured by Velvo-clear Co., Ltd.). During the measurement, the contents of the beaker are to be gently stirred to such an extent that gas bubbles are not introduced, and 100,000 biodegradable resin particles are to be measured. The volume average particle diameter of the biodegradable resin particle group is an arithmetic mean in a volume-based particle size distribution of the 100,000 particles.

<Measurement of Angle of Repose>

The biodegradable resin particle group is loosely loaded into a cylindrical frame having a diameter of 80 mm and a height of 70 mm, which is vertically placed on a horizontal surface. Next, regarding a conical heap of resin particles that is formed when the cylindrical frame is lifted, the angle formed by each of the two sides of the heap with the horizontal surface, in an elevation view of the heap, is measured with a protractor, and an average angle is determined as an average of the angles. This operation is performed five times, and an arithmetic mean of the determined average angles is designated as the angle of repose.

<Measurement of Adhesion Ratio>

A mass W1 of an artificial skin (Bioskin plate, 195 mm × 130 mm, manufactured by Beaulax Co., Ltd) is measured to the nearest 0.1 mg. Next, 0.2 g of the biodegradable resin particle group is placed on the artificial skin and uniformly spread thereon with a cell spreader, and a mass W2 of the artificial skin on which the particle group has been spread is measured. After the spreading, compressed air with a pressure of 0.05 to 0.1 MPa is blown onto the artificial skin from a position 10 cm from the artificial skin, and a mass W3 of the artificial skin is measured. An adhesion ratio is determined according to the following equation.

$$\text{Adhesion ratio (\%)} = 100 \times (W3 - W1)/(W2 - W1)$$

<Measurement of Circularity>

A flow-type particle image analyzer (FPIA®-3000S (trade name), manufactured by Sysmex Corporation) is used for the measurement.

A specific method for the measurement is as follows. 0.2 g of the biodegradable resin particle group to be measured is added to 20 ml of a 0.25% aqueous sodium dodecylbenzene sulfonate solution. The resultant is irradiated with ultrasonic waves for 5 minutes by using, as a dispersing device, a Branson Sonifier 450 ultrasonic disperser (an output of 400W and a frequency of 20 kHz), manufactured by Branson Ultrasonics, to disperse the biodegradable resin particle group in the aqueous solution of the surfactant. Accordingly, a dispersion for the measurement is prepared. The measurement is performed by using the flow-type particle image analyzer, with a standard objective lens (10×) being mounted therein. The sheath fluid to be used in the flow-type particle image analyzer is a particle sheath (PSE-900A (trade name), manufactured by Sysmex Corporation). The dispersion for the measurement,

which is prepared in accordance with the above-described procedure, is introduced into the flow-type particle image analyzer, and the measurement is performed under the following measurement conditions. For the measurement, prior to the start of the measurement, auto-focusing of the flow-type particle image analyzer is to be performed by using a suspension containing standard polymer particles (e.g., 5200A, standard polystyrene particles diluted with ion-exchanged water, manufactured by Thermo Fisher Scientific K.K.). Note that the circularity is the value obtained by dividing a circumferential length of a perfect circle, which is calculated from a diameter thereof, by a circumferential length of a biodegradable resin particle in the photographed image. The perfect circle is a perfect circle that has a projected area equal to an area of the biodegradable resin particle in the photographed image.

Measurement mode: HPF mode
Measurement range for particle diameter: 0.996 to 200 $\mu$m
Measurement range for circularity of particle: 0.5 to 1.0 $\mu$m
Number of particles to be measured: 1000

<Measurement of Mass Average Molecular Weight>

The mass average molecular weight (Mw) of the biodegradable resin particle group is a polystyrene (PS)-based mass average molecular weight measured by using a gel permeation chromatograph (GPC). Specifically, 15 mg of a sample is dissolved in 6 mL of chloroform (an immersion time of 24.0±1.0 hrs (completely dissolved)), the resultant is filtered through a 0.45-$\mu$m non-aqueous syringe filter, manufactured by Shimadzu Glc Ltd., thereafter, the measurement is performed in a chromatograph under the following measurement conditions, and the mass average molecular weight of the sample is determined from a polystyrene standard curve generated in advance.
Instrument used: a gel permeation chromatograph (equipped with an RI detector and a UV detector) HLC-8320GPC EcoSEC, manufactured by Tosoh Corporation

(GPC measurement conditions)

Column

Sample side

Guard column: TSK guard column HXL-H (6.0 mm $\times$ 4.0 cm), manufactured by Tosoh Corporation, $\times$ 1
Measurement column: TSKgel GMHXL (7.8 mm I.D. $\times$ 30 cm), manufactured by Tosoh Corporation, $\times$ 2, connected in series Reference side
Resistive tube (inside diameter of 0.1 mm $\times$ 2 m) $\times$ 2, connected in series
Column temperature: 40°C
Mobile phase: chloroform
Flow rate of mobile phase
Sample side pump: 1.0 mL/min
Reference side pump: 0.5 mL/min
Detector: RI detector or UV detector (254 nm)
Sample concentration: 0.25 wt %
Injection volume: 50 $\mu$L
Measurement time: 26 min
Sampling pitch: 500 msec

The standard polystyrene samples used for the standard curve were Standard SM-105 (product name) samples and Standard SH-75 (product name) samples, manufactured by Showa Denko K.K. These samples had mass average molecular weights of 5,620,000, 3,120,000, 1,250,000, 442,000, 151,000, 53,500, 17,000, 7,660, 2,900, and 1,320.
The standard polystyrene for the standard curve is grouped into "A" (5,620,000, 1,250,000, 151,000, 17,000, and 2,900) and "B" (3,120,000, 442,000, 53,500, 7,660, and 1,320). Subsequently, 2 mg, 3 mg, 4 mg, 4 mg, and 4 mg of the respective samples of A are weighed and subsequently dissolved in 30 mL of chloroform, and likewise, 3 mg, 4 mg, 4 mg, 4 mg, and 4 mg of the respective samples of B are weighed and subsequently dissolved in 30 mL of chloroform. The polystyrene standard curve can be obtained as follows: a measurement is performed by injecting 50 $\mu$L of each of the produced A solutions and B solutions, and a calibration curve (cubic) is generated from the obtained retention times. The standard curve is used to calculate the mass average molecular weight.

<Measurement of Ash Content>

The ash content of the biodegradable resin particle group is to be measured under the following conditions. A measurement sample of the biodegradable resin particle group is taken, and a mass W1 of the measurement sample (a mass of the measurement sample before ashing) is measured to the nearest 0.1 mg. Thereafter, 0.5 to 1.0 g of the measurement sample is added to a 30-ml magnetic crucible and heated at 750°C for 30 minutes in a microwave muffle furnace (Phoenx, manufactured by CEM corporation). Accordingly, the measurement sample is ashed, and the ashed sample is left in a desiccator to be cooled. Subsequently, a mass W2 of the ashed measurement sample (measurement sample after ashing) within the magnetic crucible is measured, and the ash content is calculated by substituting the results of W1 and W2 into the following equation.

$$\text{Ash content (mass\%)} = 100 \times W2/W1$$

<Measurement of Bulk Density>

The bulk density of the biodegradable resin particle group is to be measured in accordance with JIS K 5101-1991 (apparent density, static method).

<Measurement of Thermal Weight Loss>

The thermal weight loss of the biodegradable resin particle group is to be measured by using the following method.
9 to 10 g of the biodegradable resin particle group is weighed into a 100-ml beaker (W3) that has been dried to a constant weight, and a mass (W1) of the sample and the beaker is read to the nearest 0.1 mg. The beaker containing the taken sample is left at 150°C for 3 hours, and subsequently, the beaker is removed and allowed to stand for 30 minutes in a desiccator containing silica gel. Subsequently, a resulting mass (W2) is measured. The measurement is carried out in an environment at room temperature of 23°C to 27°C, and the thermal weight loss of the biodegradable resin particle group is calculated according to the following equation.

$$\text{Thermal weight loss (\%)} = 100 \times (W1 - W2)/(W1 - W3)$$

$W_1$: total mass (g) of the sample and the beaker before heating
$W_2$: total mass (g) of the sample and the beaker after heating
$W_3$: mass (g) of the beaker

<Method for Confirmation of Absence of Halogenated Solvents>

The confirmation of the absence of halogenated solvents in the biodegradable resin particle group is to be carried out by using the following method.

0.1 g of the biodegradable resin particles is accurately weighed into a 10-ml centrifuge tube, 5 ml of methanol is added and mixed therewith, and subsequently, the resultant is subjected to ultrasonic extraction for 15 minutes. Subsequently, centrifugation is performed at 3,500 rpm for 30 minutes, and the supernatant liquid is filtered through a 0.20-$\mu$m non-aqueous GL chromatodisc, manufactured by GL Sciences Inc. Accordingly, a sample is prepared, and the sample is examined by using the following instrument and conditions, to confirm the absence.

(GC/MS measurement conditions)

Measurement instruments: JMS-Q1000GC MkII mass spectrometer, manufactured by JEOL Ltd., and 7890A gas chromatograph, manufactured by Agilent Technologies
Column: ZB-1 capillary column (1.0 $\mu$m $\times$ 0.25 mm$\varphi$ $\times$ 60 m), manufactured by Phenomenex, Inc.
(GC oven heating conditions)
Initial temperature: 40°C (held for 3 minutes)
First phase heating rate: 15°C/min (up to 200°C)
Second phase heating rate: 25°C/min (up to 250°C)
Final temperature: 250°C (held for 14.33 minutes)
Measurement time: 30 min
Carrier gas: He
He flow rate: 1 mL/min
Injection port temperature: 250°C
Interface temperature: 250°C
Detector voltage: -900 V
Split ratio: 1/10
Ion source temperature: 250°C
Ionization current: 300 uA
Ionization energy: 70 eV
Detection method: SCAN mode (m/z = 20 to 500)

Regarding all the peaks detected on a GC/MS chromatogram of the obtained extracted sample, library search (NIST Standard Reference Database 1A, NIST/EPA/NIH MASS SPECTRAL LIBRARY (NIST 05), and NIST MASS SPECTRAL SEARCH PROGRAM Version 2.0d) is performed to confirm that no halogenated solvents are detected.

<Measurement of 3-methoxy-3-methyl-1-butanol Content>

A 3-methoxy-3-methyl-1-butanol content in the biodegradable resin particle group is to be measured by using the following method. 0.2 g of the biodegradable resin particles is accurately weighed into a 10-ml centrifuge tube, 5 ml of methanol is added and mixed therewith, and subsequently, the resultant is subjected to ultrasonic extraction for 15 minutes. Subsequently, centrifugation is performed at 3,500 rpm for 30 minutes. Subsequently, 20 $\mu$l of 1000 ppm toluene-d8 (solution in methanol), which serves as an internal standard, is added to a 2-ml volumetric flask, and the flask is made up to volume with the supernatant liquid resulting from the centrifugation. The solution, the volume of which has been made up, is filtered through a 0.20-$\mu$m non-aqueous GL chromatodisc, manufactured by GL Sciences Inc. Accordingly, a sample is prepared, and the sample is subjected to a measurement that uses the following instrument and conditions.

(GC/MS measurement conditions)

Measurement instruments: JMS-Q1000GC MkII mass spectrometer, manufactured by JEOL Ltd., and 7890A gas chromatograph, manufactured by Agilent Technologies
Column: ZB-1 capillary column (1.0 $\mu$m $\times$ 0.25 mm$\varphi$ $\times$ 60 m), manufactured by Phenomenex, Inc.
(GC oven heating conditions)
Initial temperature: 40°C (held for 3 minutes)
First phase heating rate: 15°C/min (up to 200°C)
Second phase heating rate: 25°C/min (up to 250°C)
Final temperature: 250°C (held for 6.33 minutes)
Carrier gas: He
He flow rate: 1 mL/min
Injection port temperature: 250°C
Interface temperature: 250°C
Detector voltage: -900 V
Split ratio: 1/50
Ion source temperature: 250°C
Ionization current: 300 uA
Ionization energy: 70 eV
Detection method: SIM mode (m/z = 41, 69, 91, 92, 98, 100)

In a GC/MS chromatogram of the obtained extracted sample, the area of the peak corresponding to 3-methoxy-3-methyl-1-butanol is calculated as an area ratio relative to the area of the peak of the toluene-d8, which serves as an internal standard, and the measured value is determined from a standard curve generated in advance and is designated as a 3-methoxy-3-methyl-1-butanol content in the biodegradable resin particle group.

Example 1

To a 10-L autoclave equipped with a stirring blade and a thermometer, 20 parts of polybutylene succinate (BioPBS FZ71PM, manufactured by Mitsubishi Chemical Corporation), which was used as a biodegradable resin, 30 parts of 3-methoxy-3-methyl-1-butanol (Solfit Fine Grade, manufactured by Kuraray Co., Ltd.), which was used as a solvent, 20 parts of ion-exchanged water, 35 parts of a 10% aqueous tribasic calcium phosphate solution (TCP-10U, manufactured by Taihei Chemical Industrial Co., Ltd.), which was used as a dispersion stabilizing agent, and 0.04 parts of sodium lauryl sulfate, which was used as a surfactant, were added, and the contents were stirred for 60 minutes at a heating temperature of 120°C and a stirring speed of 400 rpm. Subsequently, the mixture was cooled to 30°C over a period of 2 hours while the stirring speed was maintained. Accordingly, a suspension was obtained. 13.6 parts (1.1 times the necessary moles) of 20% hydrochloric acid was added to the obtained suspension, which was then stirred for 10 minutes to decompose the tribasic calcium phosphate. Subsequently, a biodegradable resin particle group was separated by using a centrifuge (manufactured by Tanabe Willtec Inc), and the obtained biodegradable resin particle group was washed with ion-exchanged water. The washing was completed within 1 hour after the addition of hydrochloric acid. Next, the obtained biodegradable resin particle group was dispersed in 50 parts of ion-exchanged water, and the contents were stirred with heating at 50°C for 3 hours. The resultant was cooled to 30°C to give an aqueous dispersion containing the biodegradable resin particle group. The obtained aqueous dispersion was processed in a centrifuge (manufactured by Tanabe Willtec Inc) to separate the biodegradable resin particle group, which was then washed with ion-exchanged water. Next, the obtained biodegradable resin particle group was dried for 20 hours under the conditions of 60°C and a vacuum of 0.05 MPa. Classification was performed in an air atmosphere with a relative humidity of 20%, by using a classification device (Hi-Bolter NR300 (trade name), manufactured by Toyo Hitec Co., Ltd.), equipped with a 45-$\mu$m mesh screen. Accordingly, a biodegradable resin particle group having a volume average particle diameter of 19 $\mu$m was obtained.

A thin section including a middle portion of a resin particle was cut from the biodegradable resin particle group obtained in Example 1. The thin section was stained and photographed with a transmission electron microscope (TEM).

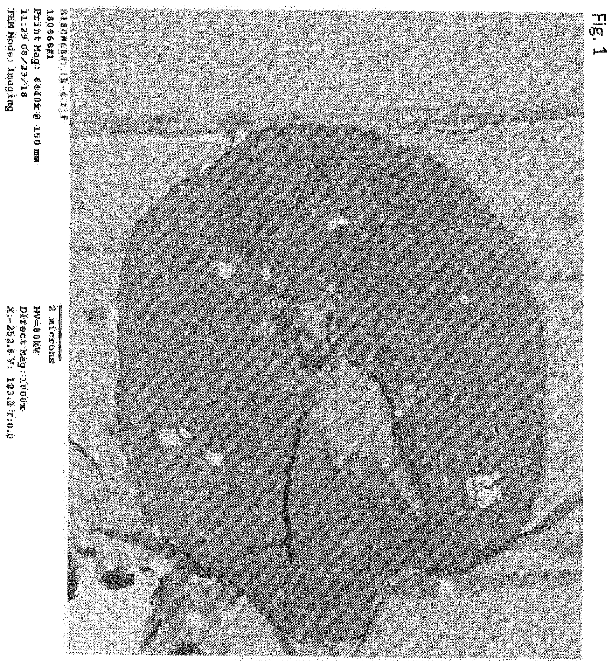

A TEM micrograph (Fig. 1) confirmed the presence of one recessed portion having a diameter of 23% and a depth of 6%, of a major diameter of the resin particle. Furthermore, the TEM micrograph (Fig. 1) confirmed the presence of two hollow portions having a diameter of 10 to 50% of the major diameter of the resin particle. In addition, TEM micrographs of ten particles having a major diameter within a range of ±50% of the volume average particle diameter were acquired. It was confirmed that nine of the ten particles had a recessed portion and a hollow portion. An average number of the recessed portions per particle and an average number of the hollow portions per particle for the ten particles are shown in Table 2. The obtained biodegradable resin particle group had an angle of repose of 63 degrees, a circularity of 0.98, a mass average molecular weight of 98,000, an ash content of 0.6%, which was an ash content after the biodegradable resin particle group was heated at 750°C for 30 minutes, a bulk density of 0.39 g/ml, a thermal weight loss of 0.5%, and a 3-methoxy-3-methyl-1-butanol content of 0.01%. No halogenated solvents were detected in the biodegradable resin particle group.

Example 2

A similar operation to that of Example 1 was performed, except that the amount of the 10% aqueous tribasic calcium phosphate solution was changed to 30 parts, and the stirring speed was changed to 250 rpm. Accordingly, a biodegradable resin particle group having a volume average particle diameter of 26 μm and a mass average molecular weight of 100,000 was obtained.

Example 3

A similar operation to that of Example 1 was performed, except that the amount of the polybutylene succinate was changed to 10 parts. Accordingly, a biodegradable resin particle group having a volume average particle diameter of 15 μm and a mass average molecular weight of 97,000 was obtained.

Example 4

To a 10-L autoclave equipped with a stirring blade and a thermometer, 5 parts of polybutylene succinate (Bio-PBS FZ71PM, manufactured by Mitsubishi Chemical Corporation), which was used as a biodegradable resin, 30 parts of 3-methoxy-3-methyl-1-butanol (Solfit Fine Grade, manufactured by Kuraray Co., Ltd.), which was used as a solvent, 20 parts of ion-exchanged water, 35 parts of a 10% aqueous tribasic calcium phosphate solution (TCP-10U, manufactured by Taihei Chemical Industrial Co., Ltd.), which was used as a dispersion stabilizing agent, and 0.04 parts of sodium lauryl sulfate, which was used as a surfactant, were added, and the contents were stirred for 60 minutes at a heating temperature of 120°C and a stirring speed of 400 rpm. Subsequently, the mixture was cooled to 30°C over a period of 2 hours while the stirring speed was maintained. Accordingly, a suspension was obtained. 13.6 parts (1.1 times the necessary moles) of 20% hydrochloric acid was added to the obtained suspension, which was then stirred for 60 minutes to decompose the tribasic calcium phosphate. Subsequently, a biodegradable resin particle group was separated by using a centrifuge (manufactured by Tanabe Willtec Inc), and the obtained biodegradable resin particle group was washed with ion-exchanged water. The washing was completed within 2 hours after the addition of hydrochloric acid. Next, the obtained biodegradable resin particle group was dried for 20 hours under the conditions of 60°C and a vacuum of 0.05 MPa. The biodegradable resin particles were classified in an atmosphere with a relative humidity of 25%, by using an air classification device (Turbo Classifier TC-15 (trade name), manufactured by Nisshin Engineering Inc.). Specifically, a swirling air flow was generated under the conditions of a rotor rotational speed of 3500 rpm and an air flow rate of 1.5 m$^3$/minute, and the biodegradable resin particles were allowed to be carried by the swirling air flow; by using an interaction of the centrifugal force imparted to the particles by the swirling air flow with the stream of the air flow toward a swirling center of the air flow, the particles were classified into particles having a large particle diameter and particles having a small diameter, and, accordingly, large particles were removed; and, accordingly, a biodegradable resin particle group having a volume average particle diameter of 9 μm and a mass average molecular weight of 95,000 was obtained.

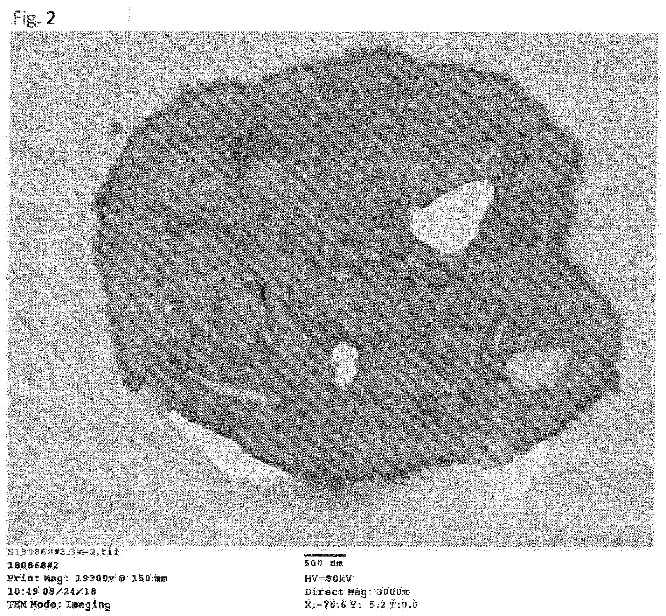

A thin section including a middle portion of a resin particle was cut from the biodegradable resin particle group obtained in Example 4. The thin section was stained and photographed with a transmission electron microscope (TEM). A TEM micrograph (Fig. 2) confirmed the presence of one recessed portion having a diameter of 33% and a depth of 7%, of a major diameter of the resin particle. Furthermore, the TEM micrograph (Fig. 2) confirmed the presence of two hollow portions having a diameter of 10 to 50% of the major diameter of the resin particle. In addition, TEM micrographs of ten particles having a major diameter within a range of ±50% of the volume average particle diameter were acquired. It was confirmed that seven of the ten particles had a recessed portion and a hollow portion. An average number of the recessed portions per particle and an average number of the hollow portions per particle for the ten particles are shown in Table 2. The obtained biodegradable resin particle group had an angle of repose of 74 degrees, a circularity of 0.97, a mass average molecular weight of 95,000, an ash content of 0.9%, which was an ash content after the biodegradable resin particle group was heated at 750°C for 30 minutes, a bulk density of 0.29 g/ml, a thermal weight loss of 2.3%, and a 3-methoxy-3-methyl-1-butanol content of 1.82%. No halogenated solvents were detected in the biodegradable resin particle group.

Example 5

A similar operation to that of Example 1 was performed, except that the polybutylene succinate was replaced with polybutylene succinate adipate (BioPBS FD92PM, manufactured by Mitsubishi Chemical Corporation). Accordingly, a biodegradable resin particle group having a volume average particle diameter of 20 $\mu$m and a mass average molecular weight of 140,000 was obtained.

Example 6

A similar operation to that of Example 1 was performed, except that the amount of the 20% hydrochloric acid added to the obtained suspension was 18.6 parts (1.5 times the necessary moles), the stirring was performed for 10 hours to decompose the tribasic calcium phosphate, and the washing was performed within 12 hours. Accordingly, a biodegradable resin particle group having a volume average particle diameter of 19 $\mu$m and a mass average molecular weight of 93,000 was obtained.

Example 7

To a 10-L autoclave equipped with a stirring blade and a thermometer, 10 parts of a 3-hydroxybutyrate/3-hydroxyhexanoate copolymer (Kaneka Biopolymer Aonilex, product number: X131A, manufactured by Kaneka Corporation), which was used as a biodegradable resin, 50 parts of 3-methoxy-3-methyl-1-butanol (Solfit Fine Grade, manufactured by Kuraray Co., Ltd.), which was used as a solvent, 5 parts of ion-exchanged water, 50 parts of a 10% aqueous tribasic calcium phosphate solution (TCP-10U, manufactured by Taihei Chemical Industrial Co., Ltd.), which was used as a dispersion stabilizing agent, and 0.02 parts of sodium lauryl sulfate, which was used as a surfactant, were added, and the contents were stirred for 60 minutes at a heating temperature of 145°C and a stirring speed of 400 rpm. Subsequently, the mixture was cooled to 30°C over a period of 2 hours while the stirring speed was maintained. Accordingly, a suspension was obtained. 17.5 parts (1.1 times the necessary moles) of 20% hydrochloric acid was added to the obtained suspension, which was then stirred for 10 minutes to decompose the tribasic calcium phosphate. Subsequently, a biodegradable resin particle group was separated by using a centrifuge (manufactured by Tanabe Willtec Inc), and the obtained biodegradable resin particle group was washed with ion-exchanged water. The washing was completed within 1 hour after the addition of hydrochloric acid. Next, the obtained biodegradable resin particle group was dispersed in 50 parts of ion-exchanged water, and the contents were stirred with heating at 50° C for 3 hours. The resultant was cooled to 30°C to give an aqueous dispersion containing the biodegradable resin particle group. The obtained aqueous dispersion was processed in a centrifuge (manufactured by Tanabe Willtec Inc) to separate the biodegradable resin particle group, which was then washed with ion-exchanged water. Next, the obtained biodegradable resin particle group was dried for 20 hours under the conditions of 60°C and a vacuum of 0.05 MPa. Classification was performed in an air atmosphere with a relative humidity of 20%, by using a classification device (Hi-Bolter NR300 (trade name), manufactured by Toyo Hitec Co., Ltd.), equipped with a 45-$\mu$m mesh screen. Accordingly, a biodegradable resin particle group having a volume average particle diameter of 19 $\mu$m was obtained.

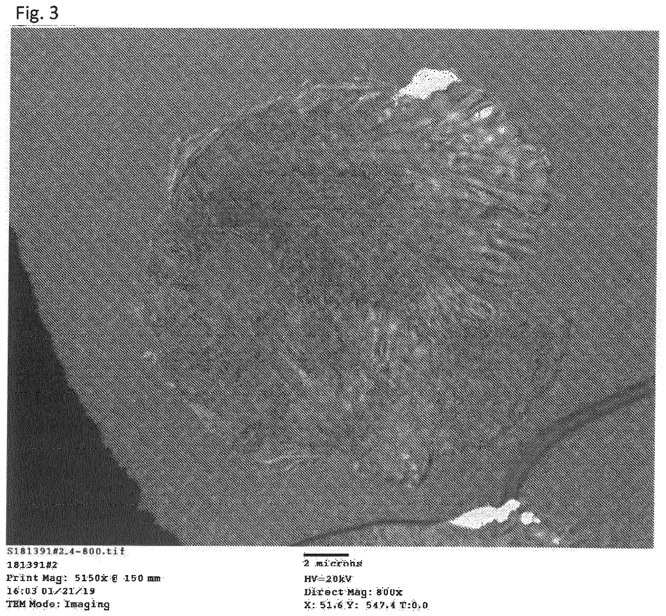

A thin section including a middle portion of a resin particle was cut from the biodegradable resin particle group obtained in Example 7. The thin section was stained and photographed with a transmission electron microscope (TEM). A TEM micrograph (Fig. 3) confirmed the presence of one recessed portion having a diameter of 28% and a depth of 12%, of a major diameter of the resin particle. Furthermore, the TEM micrograph (Fig. 3) confirmed the presence of one hollow portion having a diameter of 10 to 50% of the major diameter of the resin particle. In addition, TEM micrographs of ten particles having a major diameter within a range of $\pm$50% of the volume average particle diameter were acquired. It was confirmed that seven of the ten particles had a recessed portion and a hollow portion. An average number of the recessed portions per particle and an average number of the hollow portions per particle for the ten particles are shown in Table 2. The obtained biodegradable resin particle group had an angle of repose of 51 degrees, a circularity of 0.98, a mass average molecular weight of 391,000, an ash content of 0.4%, which was an ash content after the biodegradable resin particle group was heated at 750°C for 30 minutes, a bulk density of 0.42 g/ml, a thermal weight loss of 0.5%, and a 3-methoxy-3-methyl-1-butanol content of 0.012%. No halogenated solvents were detected in the biodegradable resin particle group.

Comparative Example 1

The measurements were performed on urethane resin particles (Plastic powder D-400, manufactured by Toshiki Pigment Co., Ltd., a volume average particle diameter of 14 μm).

Comparative Example 2

A polyethylene terephthalate resin was ground to give a polyethylene terephthalate particle group having a volume average particle diameter of 36 μm, which had a circularity of 0.83.

<Powder Characteristics Test>

The resin particles of Examples 1 to 7 and Comparative Examples 1 and 2 were evaluated by ten panelists for the feel (adhesion and spreadability) imparted by the application of the resin particles to the skin. The evaluations were made as described below based on the number of people who answered that the feel was good. A rating of "⊙" was given when the number of people was 9 to 10, a rating of "○" when the number was 7 to 8, a rating of "Δ" when the number was 4 to 6, and a rating of "×" when the number was 3 or less. The results are shown in Table 2.

(Preparation of Powder Foundation)

Mixtures including the respective resin particle groups of Examples 1 to 7 and Comparative Examples 1 and 2 were each prepared as follows: 15 parts by mass of the resin particle group, 21 parts by mass of sericite, 51 parts by mass of muscovite, 0.6 parts by mass of red iron oxide, 1 part by mass of yellow iron oxide, and 0.1 parts by mass of black iron oxide were mixed together in a Henschel mixer. Also, a solution was prepared by mixing and dissolving 1 part by mass of sorbitan sesquioleate and 0.2 parts by mass of a preservative in 10 parts by mass of cetyl 2-ethylhexanoate. The mixture and the solution were homogeneously mixed together, subsequently, 0.1 parts by mass of a flavoring agent was added thereto and homogeneously mixed, and subsequently, the resultant was ground and passed through a sieve. Accordingly, a material for a foundation was prepared. The material for a foundation was pressed into a pan, and, accordingly, a powder foundation was prepared. The ten panelists applied and spread the obtained powder foundation on their wrists and evaluated the powder foundation for adhesion to the skin and smooth spreading on the skin, according to the following criteria. The results are shown in Table 1. Note that the values in the table are averages of the test results of the ten panelists.

5: Very good
4: Good
3: Moderately good
2: Not good
1: Poor

[Table 1]

Table 1

| | Particle shape | | | Volume average particle diameter (μm) | Thermal weight loss (%) | 3-methoxy-3-methyl-1-butanol content (%) | Angle of repose (deg.) | | | Amount of adhesion (%) | Ash content (%) | Circularity | Adhesion | Smooth spreading |
|---|---|---|---|---|---|---|---|---|---|---|---|---|---|---|
| | Surface recessed portion (%) | | Hollow portion count (number) 10 to 50% diameter | | | | -0.97D + 60 | | -0.97D + 90 | | | | | |
| | diameter | depth | | | | | | | | | | | | |
| Example 1 | 23 | 6 | 2 | 19 | 0.5 | 0.010 | 42 | 63 | 72 | 58 | 0.6 | 0.98 | 4.1 | 4.4 |
| Example 2 | 9 | 2 | 3 | 26 | 0.4 | 0.009 | 35 | 58 | 65 | 51 | 0.5 | 0.98 | 3.6 | 3.8 |
| Example 3 | 19 | 4 | 2 | 15 | 0.5 | 0.021 | 45 | 69 | 75 | 69 | 0.7 | 0.98 | 4.2 | 4.3 |
| Example 4 | 33 | 7 | 2 | 9 | 2.3 | 1.820 | 51 | 74 | 81 | 93 | 0.9 | 0.97 | 4.6 | 4.0 |
| Example 5 | 20 | 6 | 1 | 20 | 0.6 | 0.013 | 41 | 61 | 71 | 55 | 0.7 | 0.97 | 4.0 | 4.1 |
| Example 6 | 23 | 6 | 2 | 19 | 0.6 | 0.016 | 42 | 64 | 72 | 59 | 0.2 | 0.98 | 4.2 | 4.0 |
| Example 7 | 28 | 12 | 1 | 19 | 0.5 | 0.012 | 42 | 51 | 72 | 51 | 0.4 | 0.98 | 3.9 | 4.1 |
| Comparative Example 1 | none | | none | 14 | - | - | 46 | 31 | 76 | 23 | 2.2 | 0.98 | 2.6 | 3.4 |
| Comparative Example 2 | none | | none | 36 | - | - | 25 | 56 | 55 | 30 | - | 0.83 | 2.5 | 4.1 |

As shown in Table 1, the powder foundations including the respective resin particle groups of Examples 1 to 7, which included a surface recessed portion and a hollow portion, were excellent in terms of both adhesion to the skin and smooth spreading on the skin, compared with the powder foundations including the respective resin particle groups of Comparative Examples 1 and 2, which included no recessed portion or hollow portion.

[Table 2]

Table 2

| | Particle shape | | | Powder characteristics test |
|---|---|---|---|---|
| | Average number of recessed portions (number) | Average number of hollow portions (number) | Abundance ratio of recessed portions and hollow portions (%) | |
| | 5 to 50% diameter and 2 to 50% depth | 10 to 50% diameter | | |
| Example 1 | 1.3 | 2.0 | 90 | ⊙ |
| Example 2 | 1.0 | 2.2 | 80 | ○ |
| Example 3 | 1.1 | 1.9 | 90 | ⊙ |
| Example 4 | 1.4 | 1.8 | 70 | ⊙ |
| Example 5 | 1.8 | 0.8 | 60 | ○ |
| Example 6 | 1.3 | 2.0 | 90 | ○ |
| Example 7 | 1.9 | 1.1 | 70 | ○ |
| Comparative Example 1 | 0 | 0 | 0 | Δ |
| Comparative Example 2 | 0 | 0 | 0 | × |

(Preparation of Milky Lotion)

2.5 parts by mass of stearic acid, 1.5 parts by mass of cetyl alcohol, 5 parts by mass of petrolatum, 10 parts by mass of liquid paraffin, and 2 parts by mass of polyethylene (10 moles) monooleic acid ester were dissolved with heating. 10 parts by mass of the biodegradable resin particle group of Example 1 was added to the resultant and mixed together, and the mixture was held at 70°C (an oil phase). Furthermore, 3 parts by mass of polyethylene glycol 1500, 1 part by mass of triethanolamine, 0.3 parts by mass of a flavoring agent, and 0.2 parts by mass of a preservative were added to 64.5 parts by mass of purified water and dissolved together with heating. The resultant was held at 70°C (an aqueous phase). The oil phase was added to the aqueous phase, the resultant was subjected to preemulsification, and subsequently, the resultant was homogeneously emulsified with a homomixer. After the emulsification, the emulsified product was cooled to 30°C with stirring, and, accordingly, a cosmetic milky lotion was obtained.

(Preparation of Solid Face Powder Cosmetic)

40 parts by mass of the biodegradable resin particle group of Example 1, 40 parts by mass of talc, 5.5 parts by mass of titanium dioxide, and a pigment were thoroughly mixed together in a kneader (a powder portion). 1 part by mass of triethanolamine was added to 50 parts by mass of purified water, and the resultant was held at 70°C (an aqueous phase). 1.5 parts by mass of stearic acid, 5 parts by mass of lanolin, 5 parts by mass of squalane, and 2 parts by mass of sorbitan sesquioleate were mixed together and then dissolved with heating and held at 70°C (an oil phase). The oil phase was added to the aqueous phase, and the resultant was homogeneously emulsified with a homomixer. The powder portion was added to the emulsified product, and the resultant was kneaded in a kneader. Subsequently, moisture was evaporated from the kneaded product, and the kneaded product was then ground in a grinding mill. In addition, a flavoring agent was uniformly sprayed onto the ground product while the ground product was thoroughly stirred, and the resultant was pressed. Accordingly, a solid face powder cosmetic was obtained.

(Preparation of Loose Powder)

5 parts by mass of the biodegradable resin particle group of Example 1, 74.6 parts by mass of talc, 12 parts by mass of synthetic phlogopite, 5 parts by mass of zinc laurate, 3 parts by mass of lauroyl lysine, and 0.4 parts by mass of iron oxide were homogeneously mixed together in a Henschel mixer. Accordingly, a loose powder was obtained.

(Preparation of Liquid Foundation)

5 parts by mass of the biodegradable resin particle group of Example 1, 11.2 parts by mass of titanium dioxide, 0.3 parts by mass of red iron oxide, 2.2 parts by mass of yellow iron oxide, and 0.2 parts by mass of black iron oxide were mixed together in a kneader (a powder portion). 5 parts by mass of isotridecyl isononanoate, 0.25 parts by mass of propylparaben, 3.5 parts by mass of dimethicone/PEG-10/15 crosspolymer, 2 parts by mass of PEG-9 polydimethylsiloxyethyl dimethicone, 20.1 parts by mass of cyclopentasiloxane, 2 parts by mass of ethylhexyl methoxycinnamate, and 2 parts by mass of disteardimonium hectorite were mixed together and then dissolved with heating at 70°C (an oil phase). 5 parts by mass of glycerol, 0.5 parts by mass of sodium chloride, 0.12 parts by mass of sodium dehydroacetate, 0.12 parts by mass of methylparaben, and 0.1 parts by mass of phenoxyethanol were added to 40.41 parts by mass of purified water and were dissolved with heating at 70°C (an aqueous phase). The powder portion was added to the oil phase, and the powder was homogeneously dispersed with a homomixer. Subsequently, the aqueous phase was added thereto, the resultant was homogeneously emulsified and dispersed with the homomixer, and subsequently, the resultant was cooled with stirring. Accordingly, a liquid foundation was obtained.

(Preparation of Pressed Powder)

8 parts by mass of the resin particle group of Example 1, 60.8 parts by mass of talc, 20 parts by mass of mica, 1.9 parts by mass of titanium dioxide, 0.14 parts by mass of red iron oxide, 0.8 parts by mass of yellow iron oxide, and 0.1 parts by mass of black iron oxide were mixed together in a Henschel mixer. Accordingly, a mixture was prepared (a powder portion). 4 parts by mass of squalane, 2 parts by mass of zinc laurate, 2 parts by mass of diisostearyl malate, 0.1 parts by mass of butyl paraben, 0.1 parts by mass of methyl paraben, 0.05 parts by mass of aluminum hydroxide, and 0.01 parts by mass of tocopherol were mixed together and then dissolved with heating at 70°C (an oil phase). The oil phase was added to the powder portion and homogeneously mixed. Subsequently, the resultant was ground and passed through a sieve, and the resultant was pressed. Accordingly, a pressed powder was prepared.

(Preparation of Eye Shadow)

5 parts by mass of the biodegradable resin particle group of Example 1, 44.1 parts by mass of talc, 20 parts by mass of mica, 10 parts by mass of mica coated with titanium dioxide, 8 parts by mass of lauroyl lysine, 2 parts by mass of zinc laurate, 0.5 parts by mass of D&C Red No. 7, and 0.4 parts by mass of FD&C Yellow No. 6 were mixed together in a kneader (a powder portion). 2 parts by mass of dimethicone and 2 parts by mass of sorbitan sesquioleate were added to 6 parts by mass of mineral oil and dissolved with heating (an oil phase). The oil phase was added to the powder portion, the resultant was kneaded in a kneader, and subsequently, the kneaded product was pressed. Accordingly, an eye shadow was obtained.

(Preparation of Skin Cream)

13 parts by mass of glycerol, 1 part by mass of decaglyceryl monostearate, 0.5 parts by mass of decaglyceryl monolaurate, 1 part by mass of glyceryl monostearate, 2 parts by mass of stearyl alcohol, 3 parts by mass of glyceryl tri(caprylate/caprate), 2 parts by mass of meadowfoam oil, 2 parts by mass of jojoba oil, 0.1 parts by mass of di(phytosteryl/octyldodecyl) lauroyl glutamate, 3 parts by mass of dimethicone, and 3 parts by mass of cyclopentasiloxane were dissolved together with heating at 70°C (an oil phase). 0.2 parts by mass of acrylates/C10-30 alkyl acrylate crosspolymer, 0.1 parts by mass of hydroxypropylmethyl cellulose, 0.05 parts by mass of disodium edetate, 0.01 parts by mass of sodium hyaluronate, 0.3 parts by mass of phenoxyethanol, 4 parts by mass of 1,3-butylene glycol, 0.1 parts by mass of sodium pyrrolidone carboxylate, and 63.1 parts by mass of purified water were dissolved together with heating at 70°C (an aqueous phase). 1 part by mass of the biodegradable resin particle group of Example 1 was added to the oil phase, the aqueous phase was then added thereto while the biodegradable resin particle group was dispersed with a homomixer, and, accordingly, a homogeneous emulsified product was obtained. 0.5 mass of a 10% aqueous sodium hydroxide solution was added to the emulsified product, and the resultant was cooled to room temperature while being stirred with a disper. Accordingly, a skin cream was obtained.

(Preparation of Body Lotion)

3 parts by mass of the biodegradable resin particle group of Example 1, 50 parts by mass of ethanol, 0.1 parts by mass of glycyrrhizic acid, 0.5 parts by mass of a flavoring agent, and 46.4 parts by mass of purified water were thoroughly mixed together in a mixer. Accordingly, a body lotion was obtained.

(Preparation of Lipstick)

10 parts by mass of the biodegradable resin particle group of Example 1, 3 parts by mass of titanium dioxide, 0.5 parts by mass of D&C Red No. 7, and 2 parts by mass of D&C Red No. 11 were added to 15 parts by mass of liquid paraffin and thoroughly mixed together in a caller (a pigment portion). 0.05 parts by mass of D&C Red No. 21 was dissolved in 11.45 parts by mass of butyl stearate (a dye portion). 12 parts by mass of ceresin, 8 parts by mass of beeswax, 5 parts by mass of cetyl alcohol, 4 parts by mass of spermaceti wax, 1 part by mass of carbana wax, 6 parts by mass of liquid paraffin, 20 parts by mass of liquid lanolin, 2 parts by mass of sorbitan sesquioleate, a flavoring agent, and an antioxidant were mixed together and then dissolved with heating. Subsequently, the pigment portion and the dye portion were added thereto and homogeneously dispersed with a homomixer. After dispersion, the resultant was poured into a mold and rapidly cooled. Accordingly, a lipstick having a stick shape was obtained.

(Preparation of Coating Material)

2 parts by mass of the biodegradable resin particle group obtained in Example 1 and 20 parts by mass of a commercially available aqueous acrylic-based gloss coating composition (Super Hit (trade name), manufactured by Kanpe Hapio Co., Ltd.) were mixed together for 3 minutes and degassed for 1 minute with a stirring/degassing device. Accordingly, a coating composition that served as a coating material was obtained. By using a coating apparatus including a blade with a clearance of 50 $\mu$m, the obtained coating composition was applied onto an ABS resin (acrylonitrile-butadiene-styrene rein) sheet and subsequently dried. Accordingly, a coating film was obtained. A gloss (60°) of the obtained coating film, which was measured by a Gloss Checker IG-330, manufactured by Horiba Ltd., was 3.

Industrial Applicability

The biodegradable resin particles of the present invention can be used in external preparations, such as cosmetics and quasi-drugs; coating materials, such as powder coating compositions and matting agents for coating compositions; rheology modifying agents; anti-blocking agents; smoothing agents; light-diffusing agents; additives for advanced ceramics sintering; fillers for adhesives; and agents for medical diagnosis and examination and can also be used by being added to a resin composition for automotive materials, construction materials, or the like or to a molded product thereof. In particular, the resin particles can be suitably used by being included in an external preparation, examples of which include cosmetics and quasi-drugs; a coating material, examples of which include powder coating compositions and matting agents for coating compositions; or an anti-blocking agent for packaging materials for food and drink or the like.

**Claims**

1. Biodegradable resin particles comprising:

    a recessed portion in a surface of the particles; and
    a hollow portion within the particles.

2. The biodegradable resin particles according to Claim 1, wherein the recessed portion has a major diameter of 5 to 50% of a major diameter of the resin particles.

3. The biodegradable resin particles according to Claim 1 or 2, wherein the recessed portion has a maximum depth of 2 to 50% of a major diameter of the resin particles.

4. The biodegradable resin particles according to any one of Claims 1 to 3, wherein the hollow portion has a major diameter of 10 to 50% of a major diameter of the resin particles.

5. The biodegradable resin particles according to any one of Claims 1 to 4, wherein a biodegradable resin is at least one resin selected from the group consisting of polyester-based resins and polyamide-based resins.

**6.** A biodegradable resin particle group comprising the biodegradable resin particles according to any one of Claims 1 to 5.

**7.** The biodegradable resin particle group according to Claim 6, wherein

ten particles having a major diameter within a range of $\pm 50\%$ of a volume average particle diameter of the biodegradable resin particle group have an average number of recessed portions per particle of 0.5 to 10 and an average number of hollow portions per particle of 0.5 to 4, and
the recessed portions are recessed portions having a major diameter of 5 to 50% and a maximum depth of 2 to 50%, of a major diameter of the resin particles, and the hollow portions are hollow portions having a major diameter of 10 to 50% of the major diameter of the resin particles.

**8.** The biodegradable resin particle group according to Claim 6 or 7, wherein the biodegradable resin particle group has a thermal weight loss of 3% or less.

**9.** The biodegradable resin particle group according to any one of Claims 6 to 8, wherein the biodegradable resin particle group has an angle of repose $\varphi$ and a volume average particle diameter D that satisfy the following formula.

$$-0.97D + 60 \leq \varphi \leq -0.97D + 90$$

**10.** The biodegradable resin particle group according to any one of Claims 6 to 9, wherein the biodegradable resin particle group has an ash content of 0.01 to 3%, and the ash content is an ash content after the biodegradable resin particle group is heated at 750°C for 30 minutes.

**11.** An external preparation, comprising the biodegradable resin particles according to any one of Claims 1 to 5 or the biodegradable resin particle group according to any one of Claims 6 to 10.

**12.** A coating material comprising the biodegradable resin particles according to any one of Claims 1 to 5 or the biodegradable resin particle group according to any one of Claims 6 to 10.

**13.** A resin composition comprising the biodegradable resin particles according to any one of Claims 1 to 5 or the biodegradable resin particle group according to any one of Claims 6 to 10.

**14.** An anti-blocking agent comprising the biodegradable resin particles according to any one of Claims 1 to 5 or the biodegradable resin particle group according to any one of Claims 6 to 10.

Fig. 1

S180868#1.1k-4.tif
180868#1
Print Mag: 6440x @ 150 mm
11:29 08/23/18
TEM Mode: Imaging

2 microns
HV=80kV
Direct Mag: 1000x
X:-252.8 Y: 123.2 T:0.0

Fig. 2

S180868#2.3k-2.tif
180868#2
Print Mag: 19300x @ 150 mm
10:49 08/24/18
TEM Mode: Imaging

500 nm
HV=80kV
Direct Mag: 3000x
X:-76.6 Y: 5.2 T:0.0

Fig. 3

S181391#2_4-800.tif
181391#2
Print Mag: 5150x @ 150 mm
16:03 01/21/19
TEM Mode: Imaging

2 microns
HV=20kV
Direct Mag: 800x
X: 51.6 Y: 547.4 T:0.0

## INTERNATIONAL SEARCH REPORT

| | International application No. |
|---|---|
| | PCT/JP2020/010757 |

**A. CLASSIFICATION OF SUBJECT MATTER**
C08J 3/16(2006.01)i; A61K 8/85(2006.01)i; A61K 8/88(2006.01)i; A61Q 1/12(2006.01)i; A61Q 19/00(2006.01)i; C08J 3/03(2006.01)i; C08L 101/16(2006.01)i
FI: C08J3/16 CFG; C08J3/03; A61K8/85; A61K8/88; A61Q1/12; A61Q19/00; C08L101/16 ZBP
According to International Patent Classification (IPC) or to both national classification and IPC

**B. FIELDS SEARCHED**

Minimum documentation searched (classification system followed by classification symbols)
C08J3/00-3/28; 99/00; A61K8/00-8/99; A61Q1/00-90/00; C08L101/16

| Documentation searched other than minimum documentation to the extent that such documents are included in the fields searched | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2017/056908 A1 (SEKISUI PLASTICS CO., LTD.) 06.04.2017 (2017-04-06) claims 1-6, 9-11, paragraphs [0002], [0007], [0012]-[0015], [0018]-[0020], [0024], [0037], [0041], [0044] | 1-14 |
| Y | claims 9-11, paragraphs [0002], [0024] | 11-14 |
| | | |
| X | WO 2010/101240 A1 (OKAYAMA UNIVERSITY) 10.09.2010 (2010-09-10) claims 1, 2, 6, 11-12, 16, paragraphs [0001], [0008], [0016], [0027]-[0028], [0034], [0036], [0066]-[0068], [0073]-[0076], fig. 1 | 1-6, 8-10 |
| Y | claim 16, paragraphs [0067]-[0068] | 11-14 |
| A | | 7 |
| | | |
| A | WO 2014/156994 A1 (SEKISUI PLASTICS CO., LTD.) 02.10.2014 (2014-10-02) entire text | 1-14 |
| | | |
| A | JP 2009-144012 A (TOHO CHEMICAL INDUSTRY CO., LTD.) 02.07.2009 (2009-07-02) entire text | 1-14 |

☒ Further documents are listed in the continuation of Box C.  ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 04 June 2020 (04.06.2020) | 16 June 2020 (16.06.2020) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

International application No.

PCT/JP2020/010757

C (Continuation). DOCUMENTS CONSIDERED TO BE RELEVANT

| Category* | Citation of document, with indication, where appropriate, of the relevant passages | Relevant to claim No. |
|---|---|---|
| A | JP 2009-120806 A (SEKISUI CHEMICAL CO., LTD.) 04.06.2009 (2009-06-04) entire text | 1-14 |
| A | JP 4-51522 A (POLA CHEMICAL INDUSTRIES, INC.) 19.08.1992 (1992-08-19) entire text | 1-14 |

Form PCT/ISA/210 (continuation of second sheet) (January 2015)

INTERNATIONAL SEARCH REPORT

Information on patent family members

| International application No. |
|---|
| PCT/JP2020/010757 |

| Patent Documents referred in the Report | Publication Date | Patent Family | Publication Date |
|---|---|---|---|
| WO 2017/056908 A1 | 06 Apr. 2017 | US 2018/0265663 A1 claims 12-18, 23-29, paragraphs [0002], [0010]-[0015], [0028]-[0034], [0038]-[0044], [0052]-[0053], [0078]-[0079], tables 1-2 EP 3357955 A1 CN 108137822 A KR 10-2018-0063076 A | |
| WO 2010/101240 A1 | 10 Sep. 2010 | (Family: none) | |
| WO 2014/156994 A1 | 02 Oct. 2014 | US 2016/0053067 A1 entire text EP 2980108 A1 KR 10-2015-0129859 A CN 105246931 A | |
| JP 2009-144012 A | 02 Jul. 2009 | (Family: none) | |
| JP 2009-120806 A | 04 Jun. 2009 | (Family: none) | |
| JP 4-51522 A | 19 Aug. 1992 | (Family: none) | |

Form PCT/ISA/210 (patent family annex) (January 2015)

29

**REFERENCES CITED IN THE DESCRIPTION**

**Patent documents cited in the description**

- JP 2017002291 A
- WO 2012105140 A
- JP 2005002302 A
- WO 2017195642 A
- WO 2013146370 A
- JP 2001139663 A